# EUROPEAN PATENT APPLICATION

(11) **EP 1 304 078 A1**
(43) Date of publication of application: **23.04.2003**
(21) Application number: 01950049.5
(22) Date of filing: 24.07.2001
(51) Int. Cl.: A61B 17/00, A61B 19/00

(54) **TISSUE SUPPORTING DEVICE FOR MEDICAL TREATMENT**

(30) Priority: 27.07.2000 JP 2000226886
(71) Applicant: JMS Co., Ltd., Hiroshima 730-8652 (JP)
(72) Inventor: KITAMURA, N., 201 4-tou, Sakyo-ku, Kyoto-shi, Kyoto 606-0842 (JP); UEMURA, Shinichi, Aso-gun, Kumamoto 869-1401 (JP)
(74) Representative: Schwarzensteiner, Marie-Luise, Dr.
(86) International application number: JP0106391
(87) International publication number: WO02009590

(57) **Abstract**

The invention provides a biomedical tissue support device for supporting, during valve repair, an unhealthy part to be excised, and performing the surgical procedure swiftly and accurately. The device includes a substantially straight rod portion (2), a curved hook portion (3) that is linked to a front end of the rod portion, and a support prop (4) connected to the hook portion's front end. The support prop has a shape that is more expanded than a connection portion to the front end of the hook portion.

## Description

### TECHNICAL FIELD

The present invention relates to biomedical tissue support devices for performing medical procedures that are used in valvoplasty for the treatment of cardiac valve disease to assist in the excision of a portion of hypertrophic tissue with an electrically driven file that rotates at high speed.

### BACKGROUND ART

Methods for treating cardiac valve disease include valve replacements in which an afflicted cardiac valve of a valvular disease patient is replaced with an artificial valve and valvoplasty in which the afflicted cardiac valve is repaired and shaped. Valve replacements have improved steadily along with advancements in artificial valves, and have reached clinical application. But even though recent surgical results for valve replacements have come to be stable, the artificial valves that are currently used are far from ideal, and considering complications arising from the artificial valve itself as well as the anticoagulation treatment that is indispensable after surgery, it hardly can be said to be a perfected therapeutic procedure. Thus, since valve replacement is not a perfected ideal procedure, it is employed as a last resort, and if possible, it is desirable that a technique that preserves the valve, such as valvoplasty (valve repair), is used as the first choice. In valvoplasty, valvulectomy with a scalpel (referred to as "slicing" in the following) has been performed conventionally, but the conventional methods were difficult with regard to the required manual skills, a promising valvoplasty was hard to achieve, and also the effect was unstable.

To address this problem, the inventors of the present invention developed a technique by which the unnecessary portion, such as a hypertrophic portion, of a biomedical tissue is excised with an electrically driven file (this is referred to as "rasping" in the following). The initial stage of this technique/method was already disclosed by the inventors in SHUJUTSU: vol. 44, No. 8, pp. 1063 to 1068 (1990), HAITOSHIN: vol. 38, No. 4, pp. 282 to 287 (1991). As the experimental trials described therein proceeded and reached clinical application, also the formation of the medial concavity of the valve cusp, which was impossible by slicing alone, became possible, and it became possible to achieve more stable results. However, problems to be solved remained also with rasping, and it is necessary to improve both the software and the hardware aspect of this technique.

One of the problems to be solved in rasping is to provide auxiliary devices that facilitate the rasping. In rasping, a portion of the hypertrophic tissue is removed by high-speed rotation of the file at the front end. When employing the rasping method, a device is necessary that supports the biomedical tissue, such as the valve, assisting in such a manner that the procedure becomes easier. That is to say, such a device is for steadily supporting the unhealthy part while the unhealthy part is excised with the rotating file, such that the excised portion does not slip away or wobble. It is necessary that such a support device can be inserted easily into the location that needs to be treated.

Furthermore, while it is necessary to excise and remove unnecessary biomedical tissue, necessary tissue must be left in place. For this reason, it is desirable to have a means for checking the wall thickness of the valve cusp, such that it is possible to excise only the portion to be removed, while checking for hypertrophic portions and thin-walled portions of the valve. However, if the measurement of the wall thickness of the valve takes too much effort and time, then it is not well suited for practical applications. Conversely, a measurement that is simple but whose precision of the wall thickness measurement is low is not suitable either. Consequently, a support device is desirable, with which the wall thickness of the valve can be checked quickly and easily, and moreover with sufficiently high accuracy.

### SUMMARY OF THE INVENTION

It is an object of the present invention to provide an effective biomedical tissue support device for facilitating surgical procedures by supporting biomedical tissue when performing rasping.

A biomedical tissue support device having a first basic configuration of the present invention supports a portion of a biomedical tissue so as to assist in a surgical procedure of excising the biomedical tissue, and comprises a substantially straight rod portion, a curved hook portion linked to a front end of the rod portion, and a support prop connected to a front end of the hook. The support prop has a shape that is more expanded than a connection portion to the front end of the hook portion.

As the rod portion is straight, it is possible to insert it easily to perform the surgical procedure even when the biomedical tissue to be excised is at a deep location. Furthermore, by holding the base end of the rod with the retractor (rib spreader), the supporting device can be arranged such that it does not cause a hindrance in the vicinity of the surgical field. The hook portion, which is curved like a fishhook, can be inserted even when the surgical field is at a narrow location, and the support prop can be positioned against the rear of the portion of the biomedical tissue to be cut away. Thus, the biomedical tissue can be supported from behind (from the rear). Furthermore, by pulling the support device carefully forward, the unhealthy part is pulled out from the deep end, so that its excision is facilitated.

The size of the support prop varies depending on application and usage, so that there are no particular limitations, but if it is used during the excision of cardiac valves, then it is preferable that the support prop has a surface area that is as large as possible, within a range that it can still be inserted through valve gaps. If a support prop is used that is more expanded than or has a wider diameter than the connection portion of the front end of the hook portion, then the tissue can be supported such that it does not slip away during the excision, and such that only the unhealthy part is excised. Furthermore, the unhealthy part can be pulled out to a location where the procedure is easily performed.

It is preferable that a maximum cross-sectional area of the support prop is larger than an a cross-sectional area of the connection portion or an average cross-sectional area of the hook portion. Here, maximum cross-sectional area of the support prop means the maximum cross-sectional area of a cross section resulting when the support prop is cut along a plane that is perpendicular to the axis of the hook portion at the front end of the hook portion. Cross-sectional area of the connection portion means the cross-sectional area resulting when the front end of the hook portion connected to the support prop is cut along a plane that is perpendicular to its (the hook portion's) axis. Furthermore, average cross-sectional area of the hook portion means the average cross-sectional area of all cross sections when cutting all portions of the hook portion along a plane that is perpendicular to the axis of the hook portion. If the cross section of the support prop and the hook portion is substantially circular, then it is also possible to take the diameter of the cross section instead of this cross-sectional area.

It is preferable that the length of the rod portion is larger than the length of the stretched out hook portion, because the unhealthy part has to be lifted up from the rear side with the support device.

It is further preferable that a light-irradiating portion of a light-projecting device that can irradiate light of an intensity that can be transmitted through the biomedical tissue is installed in a vicinity of the support prop. By irradiating light onto the excision location, it is possible to cut away the unhealthy part of the biomedical tissue while checking the wall thickness.

It is preferable that the light-projecting device includes an optical fiber having an irradiating portion at its front end, and a light-supplying portion that is connected to a base end of the optical fiber and supplies light. The support prop is made of an optically transparent material, and the rod portion and the hook portion have a continuously hollow structure. The optical fiber is inserted into an inner bore of the hook portion and the rod portion, and its one end extends from the base end side of the rode and is linked to the light-supplying portion, whereas the irradiating portion linked to the other end is held in the inner bore at the front end of the hook portion close to the support prop.

By making the support prop of an optically transparent material, the light that is irradiated from the light-irradiating portion is irradiated after passing through the irradiation face on the side opposite from the hook portion connection face. This support prop cannot only securely support biomedical tissue, but the procedure can be performed while checking the extend of the excision when excising the unhealthy part with a high-speed rotating file, and it has the advantage that it can be inserted easily into the desired location.

With the above configuration, the light-projecting device and the optical fiber, which become a hindrance during the procedure when they are outside, are inserted into the inner bore of the support device, so that they are not lying bare. Therefore, the support device can be inserted easily into the biomedical tissue. Furthermore, by letting the light be transmitted from the rear side of the biomedical tissue that has been placed on the support prop, the wall thickness at the excision location can be checked from the transmitted light, so that it is possible to prevent too much or too little excision.

A biomedical tissue support device according to a second basic configuration of the present invention comprises a substantially straight hollow rod portion, an optically transparent support prop that is fastened to a front end of the rod portion, and a light-projecting device. A surface of the support prop on the base end side of the rod constitutes a support face, and a surface area of this support face is larger than a cross-sectional area of the rod portion. The light-projecting device includes an optical fiber having an irradiating portion at its front end, and a light-supplying portion that is connected to a base end of the optical fiber and supplies light. The optical fiber is inserted into an inner bore of the rod portion, and its base end extends from the base end side of the rode and is linked to the light-supplying portion, and the irradiating portion at the front end is held in an inner bore of the support prop.

With this configuration, the inner bore of the support prop can form a substantially circular hole parallel to the rod portion and can be open to the front end side of the rod portion, and the optical fiber can extend from the front end side of the rod portion and the irradiating portion linked to this front end can be inserted into the inner bore of the support prop.

It is preferable that the support prop is of substantially circular tubular shape, and its axis is parallel to the axis of the rod portion.

It is preferable that the light-supplying portion includes an irradiation control device for controlling an irradiation intensity of light, and start / stop of the irradiation. The light-supplying portion is at a position at a distance from the irradiating portion. By including a control device with which an irradiation intensity of light, and start / stop of the irradiation can be controlled, the surgeon can turn the light irradiation manually on or off, and furthermore change the intensity of the light at the surgical field as necessary. Or, it is possible to make the color or type of the light changeable such that the biomedical tissue and its wall thickness become easy to see. It is preferable that the light-supplying portion is simple and not unwieldy, so that the surgeon can operate it manually.

It is preferable that at least a portion of the rod portion is made of a plastically deforming material.

It is also preferable that the support prop has the property that it easily transmit light of a specific direction, and does not easily transmit light of other directions.

A biomedical tissue support device according to a third basic configuration of the present invention comprises a substantially straight rod portion, and a support prop that is fastened to a front end of the rod portion. The support prop is connected to the rod portion such that it can be rotated with respect to the rod portion, and a support position located on a lateral portion of the rod portion and a stand-by position located on an extension of the front end of the rod portion can be assumed by the rotation. In the support position, a surface of the support prop on the base end side of the rod constitutes a support face, and a surface area of this support face is larger than a cross-sectional area of the rod portion.

With this configuration, the biomedical tissue support device can be inserted easily into the unhealthy part by putting the support prop in the stand-by position, and during the procedure, it can be put in the support position, so that the biomedical tissue is supported securely by the large-area support prop from the rear side of the excision location.

It is preferable that this configuration further comprises a light-projecting device. The rod portion is hollow, and the support prop is optically transparent. The light-projecting device includes an optical fiber having an irradiating portion at its front end, and a light-supplying portion that is connected to a base end of the optical fiber and supplies light. The optical fiber is inserted into an inner bore of the rod portion, and its base end extends from the base end side of the rode and is linked to the light-supplying portion, and the irradiating portion at the front end is held in an inner bore of the support prop.

It is preferable that this configuration further comprises a pull wire that is passed through an inner bore of the rod portion, an end of the pull wire being connected to the support prop. The support prop can be rotated into the support position by pulling the other end of the pull wire.

The support prop also may be biased such that a rotational force toward the stand-by position is exerted on it. Furthermore, the other end of the pull wire is connected to a control knob that is arranged near the base end of the rod portion, and configured such that a pulling force can be exerted on the pull wire with the control knob.

It is also preferable that the device comprises an auxiliary portion that is fastened to the front end of the rod portion. In the stand-by position, the support prop abuts against the auxiliary portion, and a smooth outer circumferential surface is formed by a combined body of the support prop and the auxiliary portion.

It is also preferable that the device further comprises an optical sensor for the detection of luminous energy that is installed at the rod portion and arranged above the support prop. Thus, irradiated light that has passed through the optical fiber and has been transmitted through the support prop can be detected by the optical sensor. Preferably, the optical sensor can slide on the rod portion and can be fastened to a desired position in axial direction of the rod portion.

This biomedical tissue support device can be used suitably for a procedure of partial excision of the biomedical tissue with a file rotating at high speed.

### BRIEF DESCRIPTION OF DRAWINGS

Fig. 1 is a front view showing a biomedical tissue support device according to Embodiment 1 of the present invention,
Figs. 2A to 2C are front views showing the device of Fig. 1 when it has been taken apart with a partial cross-section,
Fig. 3 is a front view showing a biomedical tissue support device according to Embodiment 2,
Fig. 4A is a top view showing an example of a support prop constituting a biomedical tissue support device in an embodiment of the present invention, and Fig. 4B is a front view thereof,
Fig. 5A is a lateral view showing another example of the support prop, and Fig. 5B is a top view thereof,
Fig. 6A is a lateral view showing another example of the support prop, and Fig. 6B is a top view thereof,
Fig. 7A is a lateral view showing another example of the support prop, and Fig. 7B is a top view thereof,
Fig. 8 is a front view showing another example of the support prop,
Fig. 9 is a front view showing another example of the support prop,
Fig. 10A is a top view showing another example of the support prop, and Fig. 10B is a lateral view thereof,
Fig. 11A is a front view showing a biomedical tissue support device in Embodiment 4 with a partial cross section, and Fig. 11B is a top view thereof,
Fig. 12A is a top view showing a support prop according to a modified example of the support device in Fig. 11, and Fig. 12B is a front view thereof,
Fig. 13A is a front view showing a rod portion in a modified example of the support device in Fig. 11, Fig. 13B is a front view thereof, and Fig. 13C is a front view of when the support prop has been assembled,
Fig. 14A is a top view showing the essential portions in a modified example of the support device in Fig. 13, Fig. 14B is a front view thereof, and Fig. 14C is a front view of another state,
Fig. 15 is a front view showing a biomedical tissue support device according to Embodiment 5,
Fig. 16 is a front view showing a biomedical tissue support device according to Embodiment 6,
Fig. 17 is a diagrammatic front view illustrating shape and dimensions of a biomedical tissue support device of the present invention,
Fig. 18A is a diagrammatic view of a prismatic support prop, taken apart, of a biomedical tissue support device in a working example of the present invention, seen from the rear side, Fig. 18B is a lateral view thereof, and Fig. 18C is a top view of that prismatic support prop taken from the top,
Fig. 19A is a rear view diagrammatically showing a bale-shaped support prop constituting a biomedical tissue support device in a working example of the present invention, and Fig. 19B is a lateral view thereof,
Fig. 20 schematically shows the gaps in a cardiac valve into which the support device of the present invention is inserted.

### BEST MODE FOR CARRYING OUT THE INVENTION

Referring to the accompanying drawings, the following is an explanation of biomedical tissue support devices for medical procedures in accordance with various embodiments of the present invention.

### Embodiment 1

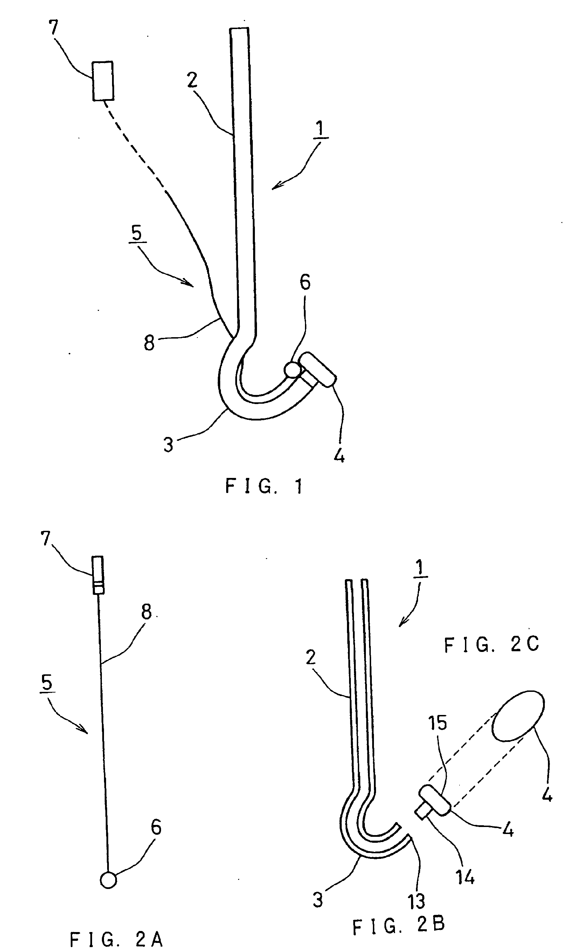

Fig. 1 shows a biomedical tissue support device for medical procedures according to Embodiment 1. This device includes a support tool 1 and a light-projecting device 5. The support tool 1 is made of a rod portion 2 shaped like a straight pole, a hook portion 3 shaped like a fishhook, and a support prop 4 that is fitted detachably to the hook portion 3. The light-projecting device 5 includes an irradiating portion 6 that irradiates light, a light-supplying portion 7 that generates and supplies light, and an optical fiber 8 for transmitting the light from the light-supplying portion 7 to the irradiating portion 6. The irradiating portion 6 is where the transmitted light is irradiated out of the optical fiber 8, and is made of a material through which light can pass. It is also possible to provide it with a light-bundling function, such as a lens. The irradiating portion 6 is mounted in the vicinity of the support prop 4.

Fig. 2 is a diagram showing the device of Fig. 1 when it has been taken apart. Fig. 2A shows the light-projecting device 5 and Fig. 2B shows the support tool 1. Fig. 2C shows the shape of an irradiation face 15, which is a surface of the support prop 4.

As shown in Fig. 2B, the support prop 4 has a fitting portion 14. The hook portion front end 13 is provided with a hollow, and the fitting portion 14 can be fitted into this hollow, thereby connecting the support prop 4 detachably and rotatably to the hook portion 3. By making the support prop 4 detachable, it becomes possible to attach or exchange support props of various shapes, as explained below, in accordance with the purpose of the procedure or the condition of the unhealthy part. Moreover, by making the support prop 4 rotatable with respect to the hook portion 3, it becomes possible to support a different location or to alter the support state of the unhealthy part. If it is preferable that the support prop 4 is not detachable or rotatable with respect to the hook portion 3, then it is also possible to make it non-detachable or non-rotatable. For example, it is possible to restrict either of those functions by providing a means for preventing rotation or a means for preventing disconnection.

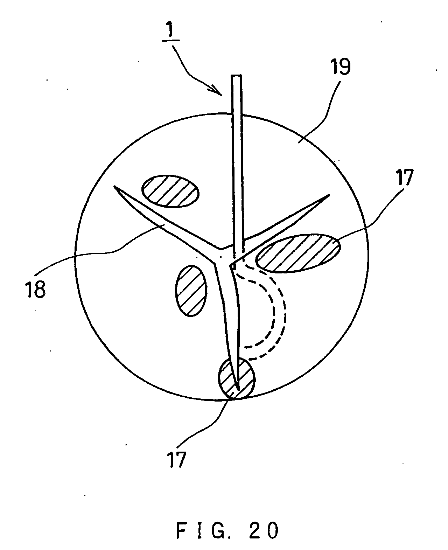

The size of the support prop 4 will depend on the application and the usage method, so that there is no particular limitation. As an example, the following is an example for a device that is preferable for usage in the excision of cardiac valves. As shown in Fig. 20, to use the support device 1, the hook portion front end is inserted through the gap 18 between the cardiac valves 19, so that with regard to easy insertion, it is preferable that the support prop 4 is small. However, when the support prop abuts against the rear side of the cardiac valve 19, and the excision location 17 on the front side is excised with the rotating file, then it does not function effectively as a presser means if the support prop is too small. Furthermore, if a large unhealthy part is excised, then the location that is supported must be changed each time, which is troublesome. Therefore, if used for the excision of cardiac valves, it is preferable that the support prop has a surface area that is as large as possible while being within a range that can be inserted through the gap 18 of the valve. If the maximum cross-sectional area of the support prop is prescribed by the ratio to the cross-sectional area of the connection portion, then it is preferable that (maximum cross-sectional area of the support prop) / (cross-sectional area of the connection portion) is in the range of 2 to 40, more preferably 5 to 20.

### Embodiment 2

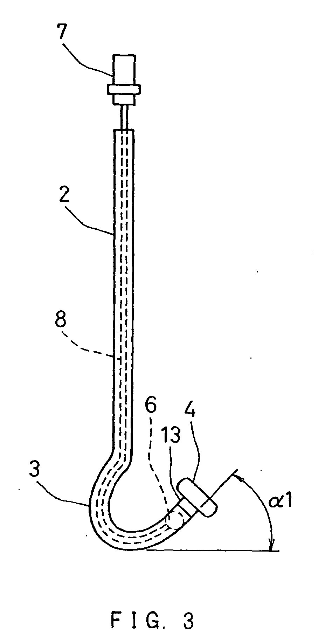

Fig. 3 shows a biomedical tissue support device according to Embodiment 2. In this embodiment, the optical fiber 8 is passed through the inner bore of the rod portion 2 and the hook portion 3, and the light-irradiating portion 6 formed at the front end of the optical fiber 8 is arranged near the hook portion front end 13. The base end of the optical fiber 8 is coupled to the light-supplying portion 7. The light-supplying portion 7 is a light source, but is also provided to function as a control means, and can turn the light irradiation on or off, and can adjust the irradiation intensity.

In order to achieve this configuration, the rod portion 2 and the hook portion 3 are provided with the hollow structure shown in Fig. 2B. The optical fiber 8, which is part of the light-projecting device 5 is inserted into their inner bores. The front end of the optical fiber 8 at which the irradiating portion 6 is formed is inserted from the base end of the rod portion 2, and when it has been brought near the support prop 4, the irradiating portion 6 is made to abut against the support prop 4 fitted onto the hook portion front end 13. The irradiating portion 6 is held in this state in the inner bore of the hook portion front end 13.

The support prop 4 is made of a material that is optically transparent. Consequently, the light that is irradiated from the irradiating portion 6 passes through the irradiation surface 15 on the side opposite to the fitting portion 14 of the support prop 4, and is irradiated.

Passing the optical fiber 8 and the irradiating portion 6 through the inner bore of the support tool 1, it is possible to irradiate light from directly below the support prop 4 while supporting the biomedical tissue with the support prop 4. Thus, light is irradiated from the rear side of the biomedical tissue that has been placed on the support prop, the wall thickness of the biomedical tissue can be checked with the transmission light that is transmitted to the surface, and the procedure can be performed while checking the degree of the excision. Furthermore, since there is no need to provide a separate space for arranging the optical fiber 8 and the irradiating portion 6, the device can be made compact and is easy to insert into the desired location. That is to say, when the light-projecting device 5 is on the outside, then it may be in the way during the surgical procedure, but if it is inserted into the inner bore of the support tool, then the support device becomes easy to insert into the biomedical tissue.

Optionally, a means for checking the wall thickness with the transmitted light may be chosen as appropriate. A check with the naked eye is the simplest and has a broad range of applications, but this is a checking means that is relative and crude. However, when a special device is necessary as a wall thickness checking means, then it becomes difficult to introduce, so that a means is desirable that is simple and with which an objective decision can be guaranteed. If the excision is performed while checking the wall thickness of the biomedical tissue with the transmitted light in this manner, then incidents due to too little or too much excision can be avoided.

If the optical transparency of the support prop 4 is devised such that light is transmitted easily only in a specific direction or at a specific surface, and not easily transmitted in other directions, then it is possible to concentrate the light only at the biomedical tissue to be excised, so that the wall thickness can be checked easily.

As described above, if the light-supplying portion 7 is at a location that is removed from the irradiating portion 6, and includes a control device with which the irradiation intensity of the light can be controlled and the light can be turned on and off, then the surgeon can turn the optical irradiation manually on or off, and can change the intensity of the light in accordance with the need for the surgical field, which is convenient. It is further possible to make the color and the type of light changeable such that the biomedical tissue or its wall thickness becomes easy to see. It is preferable that the light-supplying portion 7 is simple and not unwieldy, so that the surgeon can operate it manually. For example, as the light-projecting device, it is possible to use a device in which a cylindrical light-supplying portion, into which an AA battery has been inserted, has been connected to an optical fiber with a diameter of 2 to 3.5 mm, and an irradiating portion for irradiating the light has been formed at the front end of the optical fiber. The top of the light-supplying portion may be provided, for example, with a switch with which the intensity of the light can be controlled by turning the switch, or the irradiation can be switched on or off. The shape and the dimension of the irradiating portion and the optical fiber of the light-projecting device are designed such that they easily can be inserted into the empty support tool and retrieved therefrom.

The biomedical tissue support device of the present invention is not limited to the above-described embodiment, but for the reasons stated above, it is preferable that the optical fiber is inserted into the inner bore of the support tool. In this embodiment, the rod portion 2 and the hook portion 3 are made of a hollow metal tube. The support tool 1 is used such that it supports and pulls out the biomedical tissue during the surgical procedure. Consequently, it is disadvantageous if its stiffness is so low that it is deformed under this kind of usage. However, since it may be that the rod portion 2 is bent to adjust it to the unhealthy part or the surgical field, a portion of the rod portion 2 may have such a stiffness that it can be plastically deformed by human force. If it can be plastically deformed by stress that is smaller than that, then there is the risk that it is deformed during the surgical procedure, which is not desirable. Consequently, depending on the application, the rod portion 2 and the hook 3 can be made of stainless steel, or they can be made of aluminum or any other metal that can be plastically deformed by human force. It is also possible to adjust the weight and the plastic deformability with the wall thickness of the support tool.

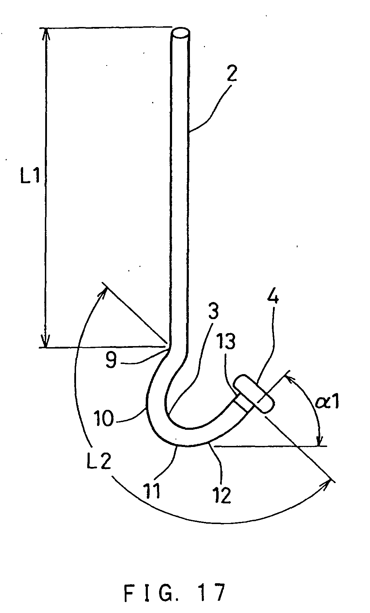

It is preferable that the length L1 of the rod portion 2 shown in Fig. 17 is longer than the length L2 of the curved hook portion stretched out to a straight line, and 2×L2 ≦ L1 ≦ 3×L2 is even more preferable. It is preferable that the support prop 4 is made of a material with excellent optical transparency, such as an acrylic resin, a polycarbonate resin, a methylmethacrylate resin or a vinylacetate resin. It is also possible to use a resin molded article with improved optical transparency of, for example, a polyvinylchloride resin, a polyolefin or a polyurethane resin.

The shape/dimensions of the rod portion 2 and the hook portion 3 shown in Fig. 3 are merely an example of the present invention, and may be changed as appropriate in accordance with the biomedical tissue to be treated or the personal preferences of the surgeon.

### Embodiment 3

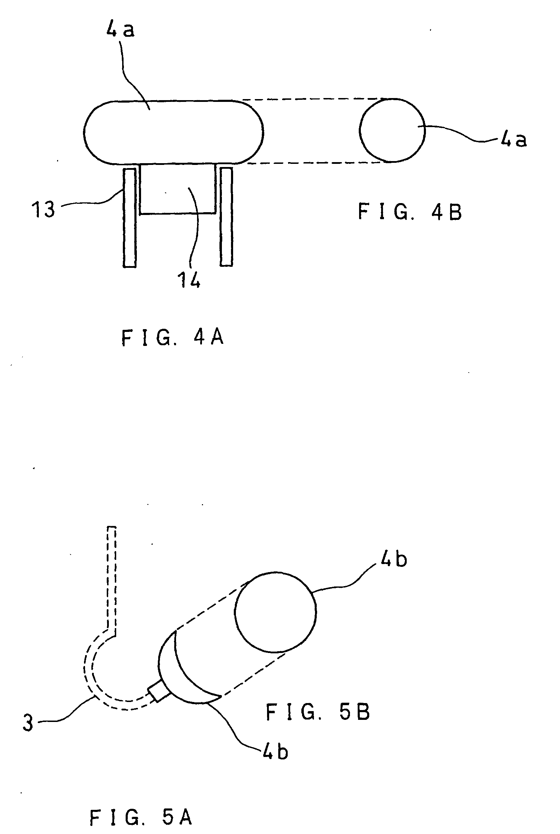

The shape of the support prop 4 shown in the above-described embodiments can be selected in accordance with purpose and object from various shapes, as shown in Fig. 4 to Fig. 10.

The support prop 4a shown in Fig. 4 has the shape of an oblate sphere (bale-shaped). Fig. 4A shows its shape seen from the front, and Fig. 4B shows its lateral shape. The support prop 4a is most commonly easy to use for the case that the lateral portion of a cardiac valve is excised. With it, a relatively wide unhealthy part can be supported such that excision is possible.

The support prop 4b shown in Fig. 5 has the shape of a bowl. Fig. 5A shows its lateral shape, and Fig. 5B shows its planar shape. This support prop 4b is suitable for use in the case that the wall thickness of the hypertrophic portion of the unhealthy part to be excised is large, and if it is circularly shaped.

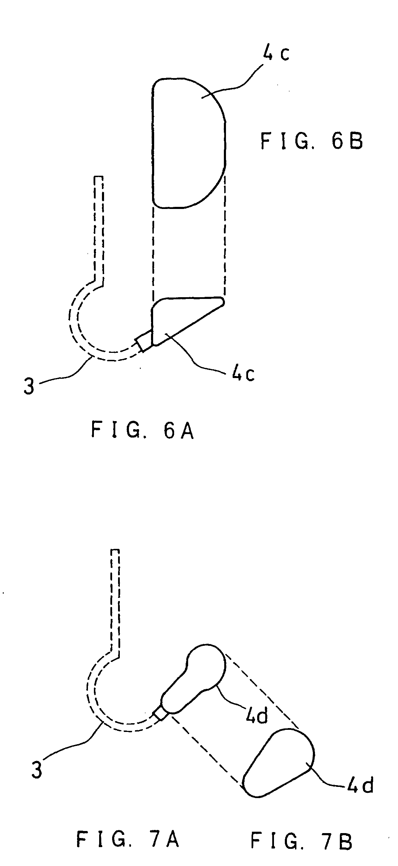

The support prop 4c shown in Fig. 6 has a prismatic shape. Fig. 6A shows its lateral shape, and Fig. 6B shows its planar shape. With this support prop 4c, support is easy even at a narrow location, when excising the end of the valve cusp.

The support prop 4d shown in Fig. 7 is pear-shaped. Fig. 7A shows its lateral shape, and Fig. 7B shows its planar shape. This support prop 4d is suitable for excision in the case that the afore-mentioned hypertrophic portion is small. This is because the risk that nearby healthy tissue is excised erroneously is small. Furthermore, this shape also can be used when the unhealthy part is of complicated shape.

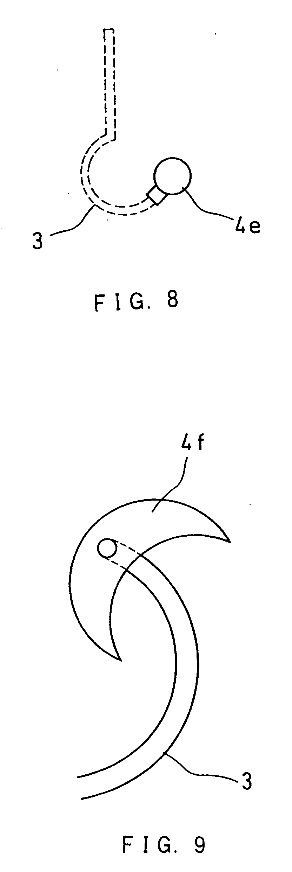

The support prop 4e shown in Fig. 8 has a simple spherical shape. This support prop 4e is suitable when the hypertrophic portion is small, or when the unhealthy part can be followed easily with the support prop.

The support prop 4e shown in Fig. 9 has the shape of an upside-down bowl. This support prop 4e is suitable for use when excising until the unhealthy part is thin.

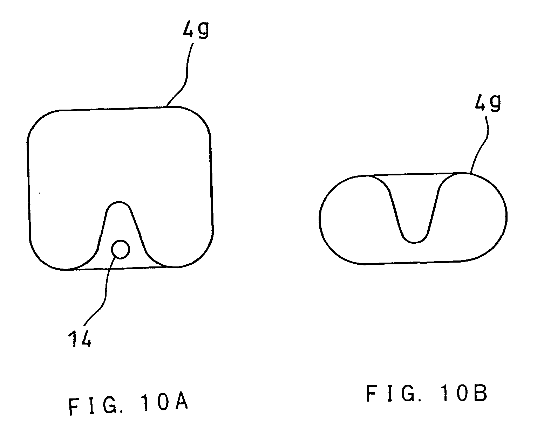

The support prop 4g in Fig. 10 is gourd-shaped. The support prop 4g is used in cases when it is not possible to follow the biomedical tissue with a support prop with a flat surface or a simple spherical surface. Fig. 10A is a top view showing the pumpkin-shaped support prop 4g from the top (the rod side), and Fig. 10B is a lateral view of the support prop 4g.

In any case, the shape and dimensions of the support prop are set such that, when excising with a file that rotates at high speed, the support prop functions as a member abutting from the rear of the excision surface, the biomedical tissue can be supported stably, and the surgical procedure can be carried out without the biomedical tissue slipping away.

### Embodiment 4

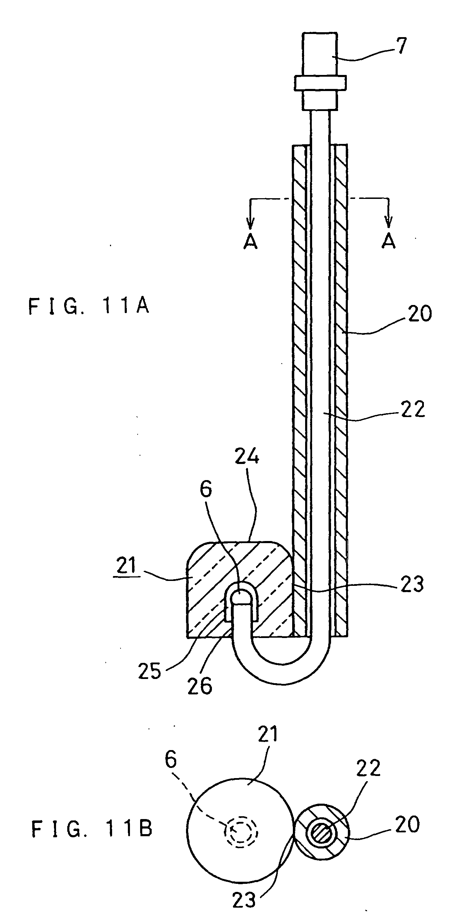

Fig. 11 shows a biomedical tissue support device according to Embodiment 4. Fig. 11A is a front view shown with a partial cross section, and Fig. 11B is a top view. It should be noted that Fig. 11B shows a state in which the top has been taken away at the cross section A·A in Fig. A.

The support tool includes a straight hollow rod portion 20 and a support prop 21 fastened to the front end thereof. The light-projecting device has a structure similar to that of Embodiment 2 shown in Fig. 3, and includes an optical fiber 22 that is inserted through the inner bore of the rod portion 20, a light-irradiating portion 6 that is formed at the front end of the optical fiber 22, and a light-supplying portion 7 connected to the base end of the optical fiber 22.

The support prop 21 is made of a prism of substantially circular cylindrical shape, and is fastened to the rod portion 20 at a circumferential surface portion 23 thereof. The central axis of the support prop 21 is parallel to the axis of the rod portion 20, and the upper face 24 of the support prop 21 is flat. Numeral 25 denotes a fiber insertion hole, and this fiber insertion hole 25 is linked to the outside by an aperture portion 26. The upper face of the support prop 21 constitutes a support face, and its surface area is larger than the cross-sectional area of the rod portion 20.

The optical fiber 22 is inserted through the aperture 26 into the fiber insertion hole 25, and the light-irradiating portion 6 at the front end is arranged at a position that is suitable for the irradiation of light. The optical fiber 22 is interlocked in the aperture portion 26 such that it cannot be removed easily from the support prop 21.

With this embodiment, the support tool can be inserted easily into the unhealthy part, and the optical fiber can be inserted easily or retrieved into or out from the support tool. Furthermore, since the structure is simple, its production is easy.

It is practical that the dimensions of the rod portion 20 are 3.5 to 8 mm outer diameter, 3 to 6 mm inner diameter, and 150 to 300 length. The wall thickness should be 0.5 to 2.0 mm, although this also depends on the material.

The upper face 24 of the support prop 21 should be large enough so that the valve ring can be supported, but it may be set as appropriate in consideration of other conditions as well. As an example of the dimensions of the support prop 21, it may be of a circular cylindrical shape of 10 to 20 mm diameter. With respect to its function, there are no particular conditions regarding thickness, and the thickness may be set as appropriate in consideration of manufacturing. Regarding the shape of the support prop 21, a circular tubular shape, the shape of a square whose corners have been beveled, or an elliptic shape are practical.

The optical fiber 22 that is used has an outer diameter of 2 to 3.5 mm and a length of at least 300 mm. The light-irradiating portion 6 also can be made by machining the front end of the optical fiber 22 and forming it in one piece therewith. It is also possible to provide no separate light-irradiating portion 6, and to use the untreated front end of the optical fiber 22 as the light-irradiating portion.

The interlocking of the optical fiber 22 with the support prop 21 may be permanent or it may be removable, and the manner of engagement can be chosen as appropriate in accordance with the way the device is used. For example, it is possible to devise the shape of the aperture portion 26 such that it fits the diameter of the light-irradiating portion 6 or the optical fiber 22, thereby achieving interlocking due to the friction force acting on the light-irradiating portion 6 or the optical fiber 22.

The length of the rod portion 20 may be chosen as appropriate, and it is preferable that a portion of about 20 mm at the front end is flexible. Alternatively, if it is devised such that it can be deformed plastically, then it can be adjusted to a suitable shape during use, which is convenient. In that case, it is preferable that it has adequate durability with respect to plastic deformation. In those cases, the entire rod portion 20 may be flexible or plastically deformable, or only the front end may be provided with those qualities by making it of a different quality.

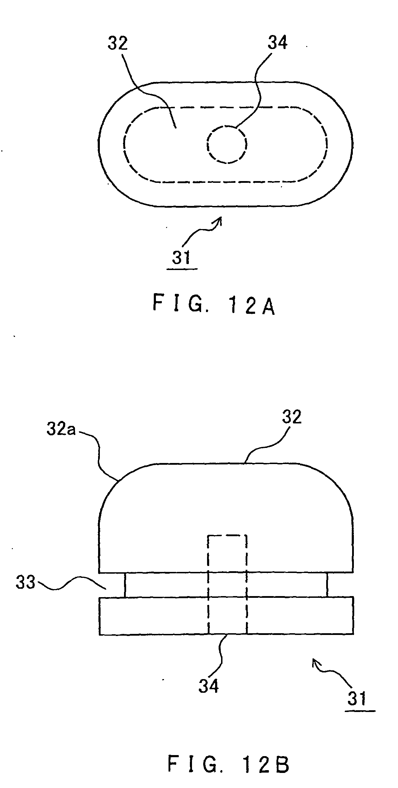

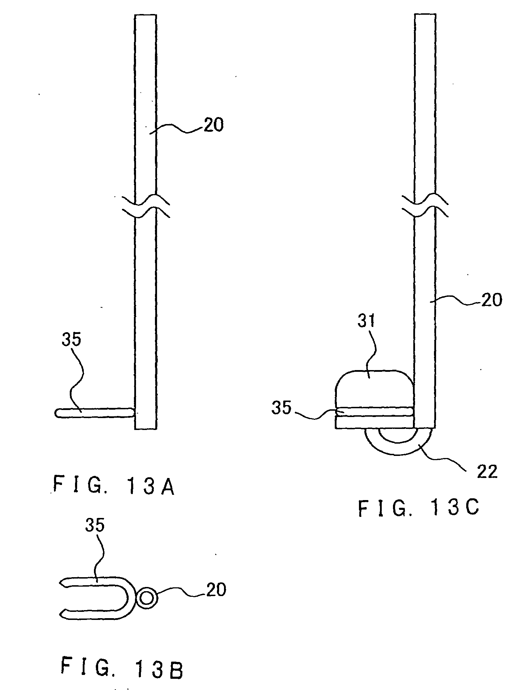

Fig. 12 and Fig. 13 show a modified example of the support device in Fig. 11. Fig. 12 shows a support prop 31 made of a prism. Fig. 12A is a top view and Fig. 12B is a front view thereof. As can be seen from the top view in Fig. 12A, the planar shape of the support prop 31 is oval. As shown in Fig. 12B, the corners of the edge portion 32a are beveled, so that the edge portion 32a is smooth. The lateral faces are provided with an engagement groove 33 for holding. The lower portion is provided with an insertion port 34 for inserting the optical fiber or the light-irradiating portion.

Fig. 13 shows a rod 20 having a prism clip 35. The support prop 31 is used by assembling it with this rod portion 20. Fig. 13A is a front view and Fig. 13B is top view. The structure of the rod portion 20 itself is similar to the one shown in Fig. 11. The planar shape of the prism clip 35 is that of an oval ring, a portion of which has been cut out, and it is fastened to the rod portion 20 by welding or the like. The shape and dimensions of the prism clip 35 are such that it can engage the engagement groove 33 for holding the support prop 31 shown in Fig. 12. Fig. 13C shows the situation when the support prop 31 is engaged by the prism clip 35. The optical fiber 22 that has been passed through the rod portion 20 is inserted in the lower portion of the support prop 31. With this structure, assembly and disassembly of the support prop 31 is easy.

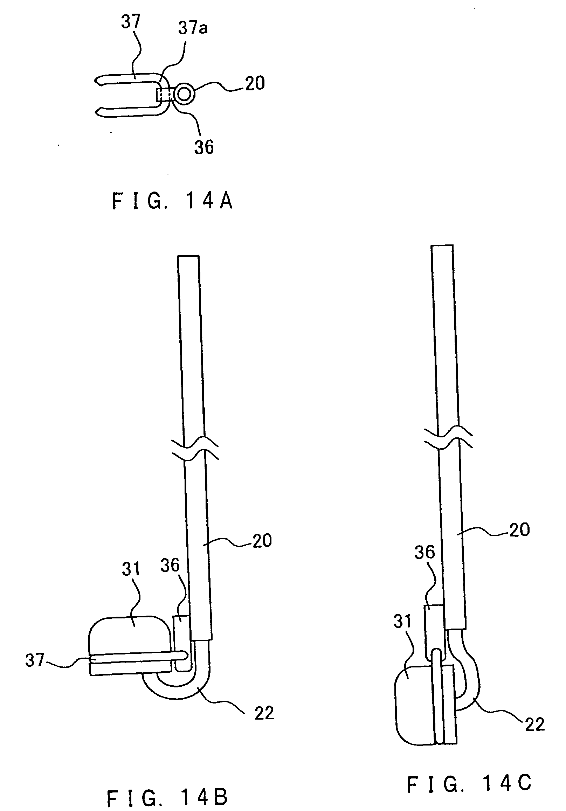

The above explanations were for an example in which the prism clip 35 is fastened to the rod portion 20, but the prism clip 35 and the rod portion 20 also may be coupled such that they can be moved with respect to one another. For example, as shown in Fig. 14A, a configuration is possible in which a bearing 36 is provided at the front end of the rod portion 20, and a linking portion 37a of a prism clip 37 is supported by that bearing 36. Thus, the bearing 36 and the linking portion 37a function as a hinge, and as shown in Fig. 14B and 14C, the support prop 31 becomes rotatable. By turning the support prop 31 from the state 14B downward for 90° to the state shown in Fig. 14C, it is possible to position the support prop 31 on an extension of the front end of the rod portion 20. In this state, the cross-sectional area at the front end portion of the support device is smaller than in the state shown in Fig. 14B. Consequently, the front end portion can be inserted easier through the gaps 18 between the cardiac valves 19 as shown in Fig. 20. Then, if the support prop 31 is returned to the state shown in Fig. 14B after the insertion, an appropriate support function can be attained.

The angle over which rotation is possible can be set as appropriate in accordance with, for example, the shape of the support prop, and accordingly also the mechanism for making rotation possible can be chosen as appropriate.

It is possible to deliver the driving force for turning the support prop 31 through the optical fiber 22. That is to say, by making the coupling between the optical fiber 22 and the support prop 31 sufficiently strong, and applying a force that pulls the optical fiber 22 from the base end of the rod portion 20, the support prop 31 is turned downward into the state shown in Fig. 14C, and by applying a force pushing it inward, it is turned in the opposite direction and restored to the state shown in Fig. 14B.

Using such a configuration enabling relative movement, it is preferable that the relative positions of the support prop 31 and the rod portion 29 can be held at least in the state shown in Fig. 14B. To this end, a mechanism interlocking the optical fiber 22 with the base end of the rod portion 20 may be provided. Alternatively, it is also possible to use any means commonly used, such as interlocking by fitting together a depressed portion and a protruding portion provided respectively in the support prop 31 and the rod portion 20.

### Embodiment 5

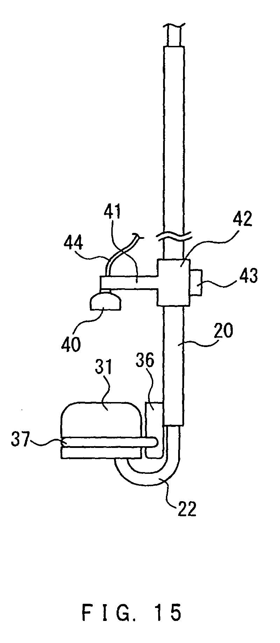

A biomedical tissue support device according to Embodiment 5 is shown in Fig. 15. This device is an example in which the device in Fig. 14 has been provided with a sensor for detecting the luminous energy that is transmitted through the biomedical tissue.

In Fig. 15, numeral 40 denotes an optical sensor for detecting luminous energy, which is supported by a coupling member 41 such that it is positioned above the support prop 31. The coupling member 41 is attached to the rod portion 20 by a fastening portion 42. The fastening portion 42 has the shape of a circular tube, and the rod portion 20 is passed through its inner bore. Consequently, the fastening portion 42 can slide with respect to the rod portion 20. Moreover, the fastening portion 42 is provided with a clamp member 43, and can be fastened at any position in axial direction of the rod portion 20. The distance between the optical sensor 40 and the surface of the support prop 31 is determined by the position at which the fastening portion 42 is fastened to the rod portion 20. The signal detected with the optical sensor 40 is sent through a cable 44 to a signal processing device (not shown in the drawings).

When using this device, the light that is transmitted through the optical fiber 22 and the support prop 31 is irradiated on the biomedical tissue placed on the support prop 31. The light that has been transmitted through the surface of the biomedical tissue is detected by the optical sensor 40, and the wall thickness of the biomedical tissue is checked based on the luminous energy. The relationship between the wall thickness of the biomedical tissue and the luminous energy detected by the optical sensor 40 has to be measured in advance. Thus, if the luminous energy of the transmitted light is detected with the optical sensor 40, it is possible perform the surgical procedure while checking the wall thickness of the biomedical tissue accurately and without necessitating expert skill.

A distance of roughly 2.3 to 4.7 cm between the light-receiving surface of the optical sensor 40 and the surface of the support prop 31 is appropriate, although this depends on various factors.

Attaching an optical sensor 40 to the biomedical tissue support device, as in this embodiment, has the advantage that the distance between the light-receiving surface of the optical sensor 40 and the surface of the support prop 31 can be kept constant during use. On the other hand, if the optical sensor 40 is arranged separately from the support device, then it is difficult to keep that distance constant. As a result, the luminous energy that is detected with the optical sensor 40 changes even if the wall thickness of the biomedical tissue is constant, and it becomes difficult to detect the wall thickness with high precision.

It should be noted that, as pointed out above, since the distance between the light-receiving surface of the optical sensor 40 and the surface of the support prop 31 is short, the optical sensor 40 may be a hindrance when performing the surgical procedure. In order to avoid this, it is preferable to adopt a structure with which relative movement of the fastening portion 42 in axial direction with respect to the rod portion 20 is prevented while allowing rotation around the axis. Thus, to detect the wall thickness, the optical sensor 40 can be rotated around the axis of the rod portion 20 and arranged above the support prop 31, and to perform the surgical procedure, it can be arranged away from the space above the support prop 31.

### Embodiment 6

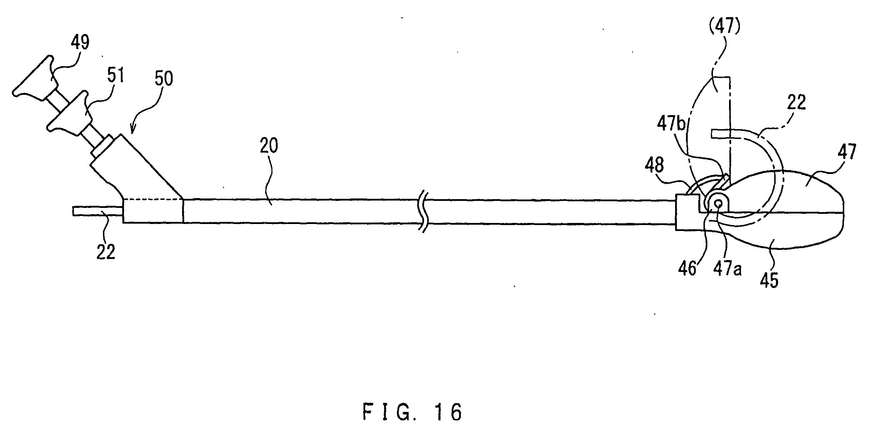

Fig. 16 is a front view of a biomedical tissue support device in accordance with Embodiment 6. In this device, an auxiliary portion 45 is fastened to the front end of the rod portion 20. This auxiliary portion 45 is provided with a bearing 46, supporting a rotation shaft 47a of the support prop 47. The support prop 47 can be rotated around the rotation shaft 47a up to the position shown by the dash-dotted line. A pull portion 47b is formed in the support prop 47, and is connected to one end of a pull wire 48.

The pull wire 48 is passed through the inner bore of the rod portion 20, like the optical fiber 22, and its other end is connected to a control knob 49. The control knob 49 is fitted into a slot 51 of a sliding guide 50, and is slidable in axial direction. By pulling the control knob 49 from the sliding guide 50, the support prop 47 is rotated counterclockwise through the pull wire 48.

Although not shown in the figures, a spring is provided in the vicinity of the rotation shaft 47a of the support prop 47, and this spring constantly exerts a clockwise spring force on the support prop 47. Consequently, if no rotational driving force is applied through the pull wire 48, the support prop 47 is pressed against the auxiliary portion 45, such that it assumes the state shown by the solid line in Fig. 16.

The planar shape of the support prop 47 may be, for example, as shown in Fig. 12 and Fig. 13. The auxiliary portion 45 is also provided with a similar planar shape. Thus, in the closed state shown by the solid line in Fig. 16, the cross-section of the support prop 47 and the auxiliary portion 45 perpendicular to the axis of the rod portion 20 becomes extraordinarily compact. Consequently, it can be inserted easily with respect to the unhealthy part.

With the biomedical tissue support device of this embodiment, the position of the support prop 47 is controlled with the pull wire 48, so that it can be controlled smoothly and without exerting stress on the optical fiber 22.

A support device as configured in the above embodiments is inserted into the unhealthy part, and the support prop is abutted against the rear side of the location of the biomedical tissue that is to be excised. Thus, the biomedical tissue is supported from the rear side of the location to be excised such that it does not slip away during the surgical procedure, and excision can be performed by an operation with a stabilized excision location. Furthermore, by configuring the rod portion and the hook portion such that they are narrower than the support prop and, if necessary, making them smooth, they can be inserted easily into the desired portion of the tissue. Thus, using the biomedical tissue support device of the present invention, excision of the biomedical tissue using a file rotating at high speed becomes easy and can be performed with precision.

The following is a specific example of shape and dimensions of a biomedical tissue support device based on the foregoing embodiments.

### Working Example 1

An example is given for the dimensions of the parts of the biomedical tissue support device of the embodiment shown in Fig. 17. The length L1 of the rod portion 2 is 150 mm, the extended length L2 of the hook portion 3 is 60 mm, and the rod portion 2 and the hook portion 3 are both made of a hollow tube of stainless steel (SUS 304, SUS 316) of φ3.5 × φ5.0. The curvature at the various portions of the hook portion 3 was set to R30 at the portion 9 linked to the rod portion 2, R25 at a first intermediate portion 10, R8 at a second intermediate portion 11, and R15 at a front end curved portion 12. The angle α1 defined by a tangent to the front end curved portion 12 and a straight line drawn out parallel to the axis of the hook portion front end 13 was set to 45°.

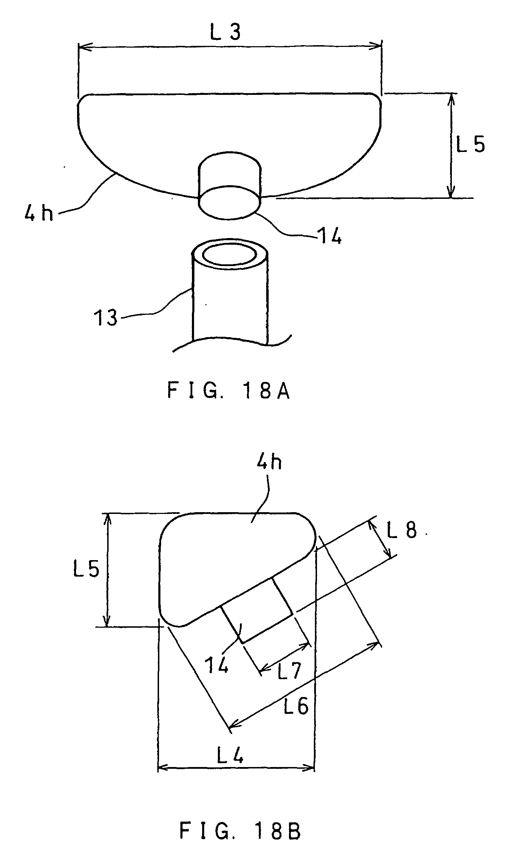

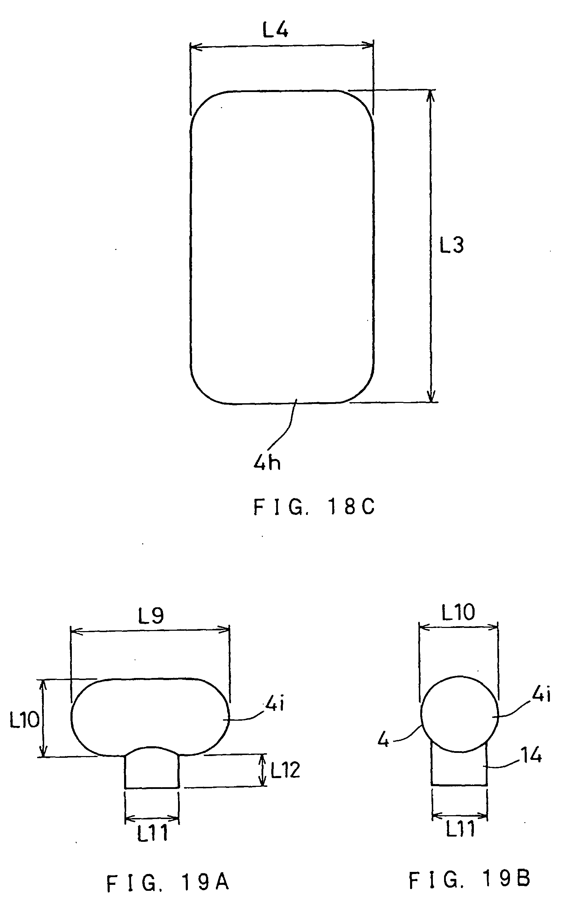

The support prop 4 fitted to the hook portion front end 13 is prismatic, as shown in Fig. 18, or bale-shaped as shown in Fig. 19.

Fig. 18A shows a prismatic support prop 4h taken from the rear. Fig. 18B is a diagram taken from the side. Fig. 18C is a top view of the support prop taken from above (on the side of the base end of the rod portion). As shown in Fig. 18A, the support prop 4h is provided with a fitting portion 14, which is fitted detachably to the hollow hook portion front end 13. The dimensions of the various portions are L3 = 20.0 mm, L4 = 10.0 mm, L5 = 7.0 mm, L6 = 22.4 mm, L7 = 3.5 mmφ, and L8 = 4.0 mm. Both the support prop 4 and the fitting portion 14 are made of acrylic resin, and have excellent optical transparency.

As another working example of the support prop, a bale-shaped support prop 4i as shown in Fig. 19 was produced. Fig. 19A is a front view, and Fig. 19B is a lateral view thereof. The dimensions of the various portions are L9 = 10.0 mm, L10 = 5.0 mm, L11 = 3.5 mmφ, and L12 = 4.0 mm.

### Working Example 2

A support device of the shape shown in Fig. 11 was produced. The length of the rod portion 20 was set to 250 mm, its outer diameter to 5.0 mm, and its inner diameter to 3.0 mm. For the light-projecting device, a known ureteral fiber was used, and the diameter of the optical fiber 22 was 2.3 mm. The prism of the support prop 21 was made detachable and exchangeable, and the long diameter of its oval planar face was set to 20 mm, the short diameter was set to 10 mm, and its height was set to 14 mm. The diameter of the fiber insertion hole 25 was set to 3.1 mm and its depth was set to 8.0 mm.

Needless to say, in the above working examples, all dimensions are examples, and the dimensions given as examples can be adjusted as appropriate.

### INDUSTRIAL APPLICABILITY

With the biomedical tissue support device of the present invention, biomedical tissue is held when performing rasping, so that the surgical procedure can be performed easily, and fast and accurate reparative surgery of biomedical becomes possible. Furthermore, the surgical procedure can be carried out while confirming the excision portion, so that a high-precision operation becomes possible that does not rely on the intuition of the surgeon.

## Claims

1. A biomedical tissue support device for medical procedures that supports a portion of a biomedical tissue so as to assist in a surgical procedure of excising the biomedical tissue, comprising a substantially straight rod portion, a curved hook portion linked to the front end of the rod portion, and a support prop connected to a front end of the hook portion, wherein the support prop has a shape that is more expanded than a connection portion to the front end of the hook portion.

2. The biomedical tissue support device for medical procedures according to claim 1, wherein a maximum cross-sectional area of the support prop is larger than a cross-sectional area of the connection portion or an average cross-sectional area of the hook portion.

3. The biomedical tissue support device for medical procedures according to claim 1, wherein a length (L1) of the rod portion is larger than a length (L2) of the stretched out hook portion (L2).

4. The biomedical tissue support device for medical procedures according to claim 1, wherein a light-irradiating portion of a light-projecting device for irradiating the biomedical tissue is installed in a vicinity of the support prop.

5. The biomedical tissue support device for medical procedures according to claim 4, wherein the light-projecting device includes an optical fiber having an irradiating portion at its front end, and a light-supplying portion that is connected to a base end of the optical fiber and supplies light, wherein the support prop is made of an optically transparent material, wherein the rod portion and the hook portion have a continuously hollow structure, wherein the optical fiber is inserted into an inner bore of the hook portion and the rod portion, and its base end extends from the base end side of the rode and is linked to the light-supplying portion, and wherein the irradiating portion at the front end is held in the inner bore at the front end of the hook portion near the support prop.

6. The biomedical tissue support device for medical procedures according to claim 1, wherein the support prop is connected detachably and/or rotatably to the hook portion.

7. The biomedical tissue support device for medical procedures according to claim 1, wherein the shape of the support prop is at least one chosen from a deformed sphere, a prismatic polyhedron, a bowl, and an upside-down bowl.

8. The biomedical tissue support device for medical procedures according to claim 1, wherein the rod portion is 50 to 200 mm long, and is at least two times longer than the stretched out hook portion.

9. A biomedical tissue support device for medical procedures that supports a portion of a biomedical tissue so as to assist in a surgical procedure of excising an unnecessary portion of the biomedical tissue, comprising a substantially straight hollow rod portion, an optically transparent support prop that is fastened to a front end of the rod portion, and a light-projecting device;
wherein a surface of the support prop on the base end side of the rod constitutes a support face, and a surface area of this support face is larger than a cross-sectional area of the rod portion; and
wherein the light-projecting device includes an optical fiber having an irradiating portion at its front end, and a light-supplying portion that is connected to a base end of the optical fiber and supplies light, wherein the optical fiber is inserted into an inner bore of the rod portion, and its base end extends from the base end side of the rode and is linked to the light-supplying portion, and wherein the irradiating portion at the front end is held in an inner bore of the support prop.

10. The biomedical tissue support device for medical procedures according to claim 9, wherein the inner bore of the support prop is provided with a substantially circular hole that is parallel to the rod portion, and is open to the front end side of the rod portion, and wherein the optical fiber extends from the front end of the rod portion, so that the irradiating portion linked to that front end is inserted into the inner bore of the support prop.

11. The biomedical tissue support device for medical procedures according to claim 9, wherein the support prop is of substantially circular tubular shape, and its axis is parallel to the axis of the rod portion.

12. The biomedical tissue support device for medical procedures according to claim 5 or 9, wherein the light-supplying portion includes an irradiation control device for controlling an irradiation intensity of light, and start / stop of the irradiation.

13. The biomedical tissue support device for medical procedures according to claim 1 or 9, wherein at least a portion of the rod portion is made of a plastically deforming material.

14. The biomedical tissue support device for medical procedures according to claim 5 or 9, wherein the support prop has the property that it easily transmit light of a specific direction, and does not easily transmit light of other directions.

15. A biomedical tissue support device for medical procedures that supports a portion of a biomedical tissue so as to assist in a surgical procedure of excising an unnecessary portion of the biomedical tissue, comprising a substantially straight rod portion, and a support prop that is fastened to a front end of the rod portion;
wherein the support prop is connected to the rod portion such that it can be rotated with respect to the rod portion, wherein a support position located on a lateral portion of the rod portion and a stand-by position located on an extension of the front end of the rod portion can be assumed by the rotation, and wherein, in the support position, a surface of the support prop on the base end side of the rod constitutes a support face, and a surface area of this support face is larger than a cross-sectional area of the rod portion.

16. The biomedical tissue support device for medical procedures according to claim 15, further comprising a light-projecting device, wherein the rod portion is hollow, and wherein the support prop is optically transparent;
wherein the light-projecting device includes an optical fiber having an irradiating portion at its front end, and a light-supplying portion that is connected to a base end of the optical fiber and supplies light, wherein the optical fiber is inserted into an inner bore of the rod portion, and its base end extends from the base end side of the rode and is linked to the light-supplying portion, and wherein the irradiating portion at the front end is held in an inner bore of the support prop.

17. The biomedical tissue support device for medical procedures according to claim 15 or 16, wherein the rod portion is hollow, further comprising a pull wire that is passed through an inner bore of the rod portion, an end of the pull wire being connected to the support prop, wherein the support prop is rotated into the support position by pulling the other end of the pull wire.

18. The biomedical tissue support device for medical procedures according to claim 17, wherein the support prop is biased such that a rotational force toward the stand-by position is exerted on it.

19. The biomedical tissue support device for medical procedures according to claim 17, wherein the other end of the pull wire is connected to a control know that is arranged near the base end of the rod portion, and configured such that a pulling force can be exerted on the pull wire with the control knob.

20. The biomedical tissue support device for medical procedures according to claim 17, further comprising an auxiliary portion that is fastened to the front end of the rod portion, wherein in the stand-by position, the support prop abuts against the auxiliary portion, and a smooth outer circumferential surface is formed by a combined body of the support prop and the auxiliary portion.

21. The biomedical tissue support device for medical procedures according to claim 9, further comprising an optical sensor for detecting luminous energy that is installed at the rod portion and arranged above the support prop, wherein irradiated light that has passed through the optical fiber and has been transmitted through the support prop can be detected by the optical sensor.

22. The biomedical tissue support device for medical procedures according to claim 21, wherein the optical sensor can slide on the rod portion and can be fastened to a desired position in axial direction of the rod portion.

23. The biomedical tissue support device for medical procedures according to claim 1 or 9, used for a surgical procedure of partial excision of the biomedical tissue with a file rotating at high speed.
